(19) Europäisches Patentamt
European Patent Office
Office européen des brevets

(11) Veröffentlichungsnummer: **0 035 121**
**B1**

(12) # EUROPÄISCHE PATENTSCHRIFT

(45) Veröffentlichungstag der Patentschrift: **19.12.84**

(51) Int. Cl.³: **C 12 N 11/04**, C 12 N 11/08, C 12 N 11/12

(21) Anmeldenummer: **81100722.8**

(22) Anmeldetag: **02.02.81**

(54) **Verfahren zur Herstellung von perlförmigen Biokatalysatoren mit extrem empfindlicher enzymatisch aktiver Substanz.**

(30) Priorität: **15.02.80 DE 3005633**

(43) Veröffentlichungstag der Anmeldung:
**09.09.81 Patentblatt 81/36**

(45) Bekanntmachung des Hinweises auf die Patenterteilung:
**19.12.84 Patentblatt 84/51**

(84) Benannte Vertragsstaaten:
**AT BE CH FR GB IT LI LU NL SE**

(56) Entgegenhaltungen:
**EP-A-0 034 293**

**CHEMICAL ABSTRACTS, Band 90, Nr. 21, 21. Mai 1979, Seite 221, Nr. 164051b Columbus, Ohio, U.S.A.**
**CHEMICAL ABSTRACTS, Band 90, Nr. 21, 21. Mai 1979, Seite 221, Nr. 164052c Columbus, Ohio, U.S.A.**

**Die Akte enthält technische Angaben, die nach dem Eingang der Anmeldung eingereicht wurden und die nicht in dieser Patentschrift enthalten sind.**

(73) Patentinhaber: **Klein, Joachim, Prof. Dr.**
**Hühnerkamp 21**
**D-3300 Braunschweig (DE)**
(73) Patentinhaber: **Wagner, Fritz, Prof. Dr.**
**Hohe Wiese 2**
**D-3300 Braunschweig-Stöckheim (DE)**

(72) Erfinder: **Klein, Joachim Prof.Dr.**
**Hühnerkamp 21**
**D-3300 Braunschweig (DE)**
Erfinder: **Wagner, Fritz Prof.Dr.**
**Hohe Wiese 2**
**D-3300 Braunschweig-Stöckheim (DE)**
Erfinder: **Vorlop, Klaus-Dieter Dipl.-Chem.**
**Hochstrasse 7**
**D-3300 Braunschweig (DE)**

(74) Vertreter: **Jahn-Held, Wilhelm W., Dr.Dr.-Ing. Dipl.Chem.**
**Schöne Aussicht 8**
**D-3513 Staufenberg 1 (DE)**

(56) Entgegenhaltungen:
**CHEMICAL ABSTRACTS, Band 90, Nr. 21, 21. Mai 1979, Seite 221, Nr. 164053d Columbus, Ohio, U.S.A.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 035 121 B1

CHEMICAL ABSTRACTS, Band 81, Nr. 6, 12. August 1974, Seite 111, Nr. 27385p Columbus, Ohio, U.S.A. M.S. MASRI et al.: "Binding of metal cations by natural substances"
DIE ANGEWANDTE MAKROMOLEKULARE CHEMIE 76/77, 1979, Seiten 329-350 J. KLEIN et al.: "Polymernetzwerke zum Einschluss von Mikroorganismen"

**Beschreibung**

Die Fixierung von Enzymen, Zellfragmenten und ganzen Zellen hat in jüngster Zeit in der Fachwelt besondere Aufmerksamkeit gefunden. Dies liegt daran, dass solche Verfahren für die industrielle, biokatalytische Produktion von technischer und wirtschaftlicher Bedeutung ist.

Der Stand der Technik beschreibt in Chemical Abstracts Verfahren, die sich in den Einsatzstoffen und in den Verfahrenmassnahmen von dem Verfahren der Erfindung unterscheiden.

Chemical Abstracts, Bd. 90, Nr. 164051, b bebschreibt für Glukose-Isomerase-produzierende Mikroorganismen eine Entwässerung, Granulierung und Trocknung bei 60°C der gefällten Zellen.

Chemical Abstracts, Bd. 90, Nr. 164052, c beschreibt für solche Zellen nach der Filtrierung und Granulierung eine Trocknung mit heisser Luft bei 80°C und danach bei 30°C über die Nacht durch.

Chemical Abstracts, Bd. 90, Nr. 164053, d beschreibt für solche Zellen ebenfalls die Trocknung mit heisser Luft bei 70°C.

Ein Verfahren zur Herstellung von Biokatalysatoren mit hoher mechanischer Festigkeit und hoher Beladung an enzymatisch aktiver Substanz durch Polymereinschluss von ganzen Zellen, Zellfragmenten oder Enzymen ist Gegenstand der deutschen Patentschrift 28 35 875.

Dieses Verfahren betrifft die Herstellung einer Suspension oder Lösung (A) einer enzymatisch aktiven Substanz in der wässrigen Lösung eines Polyelektrolyten und eines Fällungsbades (B) aus einer Lösung einer Verbindung, die mehrwertige, dem Polyelektrolyten der Mischung (A) entgegengesetzt geladenen Ionen, enthält. Es werden aus den Mischungen (A) und (B) durch Eindüsen von (A) in (B) perlförmige Teilchen im Kornbereich von etwa 0,5 bis 5 mm hergestellt, die an ihrer Oberfläche etwa 5 min bis 4 h verfestigt werden, daraus wird in Stufe 2 durch Waschen mit physiologischer Salzlösung und in Stufe 3 durch schonende Trocknung bis 80°C bis etwa 48 h in in Stufe 4 durch Eintragen in ein Fällungsbad der Mischung (B) und durch einen Waschprozess in Stufe 5 die Biokatalysatoren hergestellt.

Die nach diesem Verfahren hergestellten Biokatalysatoren-Perlen weisen eine hohe mechanische Festigkeit und eine hohe Beladung an enzymatisch aktiver Substanz auf. Die Druckfestigkeit solcher Katalysatorenperlen weisen eine Druckfestigkeit (P/Perle) von über 800 bis 1000 und eine Beladung in Gramm enzymastischer Substanz pro Gramm Katalysatorperlen bis 0,9 auf. Es können bereits Werte über 0,2 als höher als der bisher bekannte Stand der Technik bezeichnet werden.

Das Verfahren der deutschen Patentanmeldung DE—A—3005632 zur Herstellung von Biokatalysatoren mit hoher mechanischer Festigkeit und hoher Beladung an enzymatisch aktiver Substanz durch Polymereinschluss von ganzen Zellen, Zellfragmenten oder Enzymen ist dadurch gekennzeichnet, dass in Stufe 1 die Mischung (A), welche einen kationischen Polyelektrolyten im Konzentrationsbereich von 0,1 bis 15 Gew.-% enthält, in die Mischung (B) mit einem, ein mehrwertiges Anion enthaltenden Verbindung, im Konzentrationsbereich von 0,02 bis 35 Gew.-% eingedüst wird unter Bildung perlförmiger Teilchen eines Kornbereiches von etwa 0,5 bis etwa 10 mm und diese unter Rühren in einer Zeit zwischen etwa 0,5 bis 4 h verfestigt werden, und in Stufe 2 die Katalysatorperlen mit der eingeschlossenen enzymatischen Substanz abfiltriert, mit Wasser gewaschen oder einer wässrigen Salzlösung gewaschsen und in Stufe 3 durch überleiten von Luft einer Temperatur bis 80°C bis etwa 48 h unter Schrumpfung und Verfestigung getrocknet werden und in Stufe 4 die Katalysatorperlen zur Äquilibrierung in eine, das Enzym-System und das Netzwerk stabilisierende, wässrige Lösung eingebracht und danach im feuchten Zustand bis nach etwa 24 h ausgeführt werden.

Nach dem Verfahren gemäss der Erfindung liegt erstmalig eine ionotrope Matrix vor, die in der Lösung eines Phosphatpuffers stabil ist. Dieses Verfahren wird durch das Verfahren der Erfindung erweitert.

Das Verfahren der Erfindung stellt sich die Aufgabe, nun auch hochempfindliche Enzyme oder Enzym-Systeme, insbesondere lebende Zellen, zu immobilisieren und in einer Phosphat-Pufferlösung aktiv zu erhalten.

Diese erweiterte Aufgabe kann durch die Verfahren nach dem nicht vorveröffentlichten Stand der Technik nicht gelöst werden. Die einfache Vernetzung mit Alginat, auch ohne Härtung, kann diese Aufgabe der Technik nicht lösen, weil die Matrix sich in einer Phosphat-Pufferlösung wieder auflöst.

Dazu wird auf S Suzuki und J. Karube in Immobilized Microbial Cells, Seite 60, hingewiesen.

Es ist zwar durch Verwendung des kationischen Polyelektrolyten "Chitosan" möglich, diese Auflösung zu verhindern, aber es ist die Verfahrensstufe der Trocknung der Katalysatorperlen auf hochempfindliche Systeme nicht anwendbar. Dieser Nachteil wird durch das Verfahren der Erfindung überwunden und dieser Stand der Technik auf solche hochempfindlichen Systeme erweitert.

Zur Lösung dieser Aufgabe des Verfahrens der Erfindung wird in Stufe 1 die Mischung (A) als Lösung oder Suspension einer enzymatisch aktiven Substanz in der wässrigen Lösung eines kationsichen Polyelektrolyten in einem Konzentrationsbereich von 0,1 bis 15 Gew.-% in die Mischung (B) als Fällungsbad aus der Lösung eines mehrwertigen Anions im Temperaturbereich von —5°C bis 80°C eingedüst unter Entstehung perlförmiger Teilchen mit einem bestimmten Kornbereich von etwa 0,5 bis etwa

10 mm und diese unter Rühren innerhalb einer Verweilzeit von etwa 5 min bis 4 b verfestigt und danach in Stufe 2 die gebildeten Katalysatorperlen mit der eingeschlossenen enzymatisch aktiven Substanz abfiltriert und zur Äquilibrierung in eine, das Enzym-System und das Netzwerk stabilisierende, wässrige Lösung eingebracht und danach im feuchten Zustand ausgeführt.

Das Verfahren der Erfindung ist im Patentanspruch 1 definiert.

Die alternative Ausgestaltung des Verfahrens der Erfindung ist in den weiteren Ansprüchen definiert.

Diese betreffen die enzymatisch aktive Substanz aus vermehrungsfähigen, ganzen Zellen oder Zellfragmenten oder aus einem isoliertem Enzym oder einem Enzym-Komplex, vorzugsweise aus gegen mehrwertige Kationen empfindlichen oder mechanisch empfindlichen oder temperaturempfindlichen oder trocknungsempfindlichen Zellen, Zellfragmenten oder Enzymen. Diese betreffen auch die Mischung (A) in die als kationischer Polyelektrolyt ein natürliches Polymer oder ein natürliches Polymer nach chemischer Modifizierung eingesetzt wird, oder auch ein synthetischer kationischer Polyelektrolyt und zwar solche, die ionisch vernetzbar sind.

Diese betreffen auch die Mischung (B), in welcher als ionische Verbindung mit mehrwertigem Anion die Verbindungen

$$K_4\ (Fe(CN)_6)\ \text{und/oder}\ K_3(Fe(CN)_6)$$

oder ein mehrwertiges Polyphosphat mit einem Polymergrad kleiner 15 eingesetzt werden.

Diese betreffen auch die Stufe 2, in welcher zur Äquilibrierung des Enzym-Systems und des Netzwerkes eine Phosphat-Pufferlösung eingesetzt wird.

Das Verfahren der Erfindung wird durch das folgende Ausführungsbeispiel beschrieben. Dieses ist jedoch nicht auf dieses Beispiel beschränkt.

Beispiel

Es wird die Mischung (A) durch Suspension von 2,5 g Pseudomonas 3ab DSM 672 in Form der zentrifugierten feuchten Masse in 27,5 g einer 2,0 Gew.-%igen, wäßrigen Chitosanacetat-Lösung als Polyelektrolyt hergestellt. Als Fällungsbad (B) mit der Verbindung, die mehrwertige Ionen enthält, die dem Polyelektrolyten der Mischung (A) entgegengesetzt geladen sind, wird eine 0,05 molare $K_4(Fe(CN)_6)$—Lösung verwendet.

Es wird dann in Stufe 1 in die Mischung (B) eingetropft, wobei perlförmige Teilchen mit einem Durchmesser von ca. 5 mm entstehen. Diese werden unter weiterem Rühren bei einer Verweilzeit von 45 Minuten verfestigt. Es werden danach in Stufe 2 die gebildeten Katalysatorperlen mit der eingeschlossenen enzymatisch aktiven Substanz abfiltriert. Es werden danach die Perlen zur Aquilibierung in eine 0,1 molare K-Phosphat-Pufferlösung mit pH 8,5 für einen Zeitraum von 3 Stunden gegeben.

Unter Einsatz dieser, nach dem Verfahren der Erfindung erzeugter Biokatalysatorperlen ist es möglich, die Synthese von Serin aus Glycin und Methanol nach dem Verfahren der DE—AS 2 554 530 durchzuführen mit einer relativen Aktivität von 15%. Diese relative Aktivität ergibt sich aus dem Vergleich des Umsatzes, der sich nach 8 h mit jeweils 2 g BFM in freiem und immobilisierten Zustand in 100 ml Testlösung (10 g/l Glycin; 0,8% Methanol) einstellt. Dabei betrug die Temperatur 30°C und es wurde in einem Medium nach Jayasuria, Journ. Chem. Microbial. 12 (1955), 419, bei einem pH-Wert von 8,5 gearbeitet.

Das Verfahren der Erfindung löst die erweiterte Aufgabe der Anwendung auf hochempfindliche Systeme unter Erhaltung der Aktivität.

Es wird zur Lösung der Aufgabe der Erfindung auf die hohe Beladung und die hohe Festigkeit der Biokatalysatoren gemäss der Erfindung verzichtet, die durch den Trocknungsprozess erzielt wird.

Die katalytische Aktivität der Pseudomonas-Zellen nach dem Beispiel war nach dieser Trocknunggstufe auf den Wert Null abgesunken.

Es hat sich überrachend gezeigt, dass auch ohne diese Trocknungsstufe eine zwar niedrigere, aber in der Praxis noch ausreichende Festigkeit der Biokatalysatorperlen erzielt wird.

Die Katalysatorperlen nach dem Verfahren gemäss der Erfindung sind offenbar aufgrund ihrer Elastizität auch bei intensivem Rühren stabil. Es ist deshalb überraschend möglich, eine biotechnische Synthese mit derartigen, hochempfindlichen Enzymen oder Enzym-Systemen durchzuführen. In dieser Erweiterung der erfindungsgemäss hergestellten Biokatalysatoren auf hochempfindliche Systeme liegt ein für die Biotechnologie erheblicher technischer Vorteil.

## Patentansprüche

1. Verfahren zur Herstellung perlförmiger Biokatalysatoren mit extrem empfindlicher, enzymatisch aktiver Substanz durch ionische Vernetzung von Polykationen unter Verwendung der Mischung (A) als Lösung oder Suspension einer enzymatisch aktiven Substanz in der wässrigen Lösung eines Polyelektrolyten, dadurch gekennzeichnet, dass in der Mischung (A) als kationischer Polyelektrolyt ein protoniertes Chitosan oder ein natürliches Polymer oder ein natürliches Polymer nach chemischer Modifizierung oder ein synthetischer kationischer Polyelektrolyt in einem Konzentrationsbereich von 0,1 bis 15 Gew.-% eingesetzt wird, und als Fällungsbad die Lösung eines mehrwertigen Anions im Temperaturbereich von −5°C bis 80°C als Mischung (B) verwendet wird, und in Stufe 1 die Mischung (A) in die Mischung (B) eingedüst wird unter Bildung perlförmiger Teilchen eines Kornbereiches von etwa 0,5 bis etwa 10 mm, und diese unter Rühren innerhalb einer Verweilzeit von etwa 5 min bis 4 h verfestigt werden, und in Stufe 2 die gebildeten Katalysatorperlen mit der eingeschlossenen, enzymatisch aktiven Substanz abfiltriert und zur Äquilibrierung in eine, das Enzym-System und das Netzwerk stabilisierende, wässrige Lösung eingebracht und danach im feuchten Zustand ausgeführt werden.

2. Verfahren nach Anspruch 1, dadurch gekennzeichnet, dass das Fällungsbad (B) einen Konzentrationsbereich von 0,02 bis 35% aufweist.

3. Verfahren nach den Ansprüchen 1 und 2, dadurch gekennzeichnet, dass die enzymatisch aktive Substanz aus vermehrungsfähigen, ganzen Zellen oder nicht vermehrungsfähigen ganzen Zellen oder Zellfragmenten besteht und als Suspension eingesetzt wird.

4. Verfahren nach den Ansprüchen 1 bis 3, dadurch gekennzeichnet, dass die enzymatisch aktive Substanz aus einem isolierten Enzym oder einem Enzym-Komplex besteht und als Lösung oder Suspension eingesetzt wird.

5. Verfahren nach den Ansprüchen 1 bis 4, dadurch gekennzeichnet, dass als enzymatisch aktive Substanz gegen mehrwertige Kationen empfindliche oder mechanisch empfindliche oder temperaturempfindliche oder trocknungsempfindliche Zellen, Zellfragmente oder Enzyme unter Beibehaltung physiologischer, osmotischer Bedingungen, eingesetzt werden.

6. Verfahren nach den Ansprüchen 1 bis 5, dadurch gekennzeichnet, dass in Mischung (A) als kationischer Polyelektrolyt ein natürliches Polymer oder ein natürliches Polymer nach chemischer Modifierung eingesetzt wird.

7. Verfahren nach den Ansprüchen 1 bis 6, dadurch gekennzeichnet, dass in Mischung (A) ein synthetischer kationischer Polyelektrolyt eingesetzt wird.

8. Verfahren nach den Ansprüchen 1 bis 7, dadurch gekennzeichnet, dass in Mischung (A) als kationischer Polyelektrolyt ein protoniertes Chitosan in einem Konzentrationsbereich von 0,1 bis 15 Gew.-% eingesetzt wird.

9. Verfahren nach den Ansprüchen 1 bis 8, dadurch gekennzeichnet, dass in Mischung (B) als ionische Verbindung mit mehrwertigem Anion

$$K_4(Fe(CN)_6) \text{ und/oder } K_3(Fe(CN)_6)$$

in einem Konzentrationsbereich von 0,006 bis 1 Mol/l eingesetzt wird.

10. Verfahren nach den Ansprüchen 1 bis 9 dadurch gekennzeichnet, dass als Lösung in Stufe 2 zur Äquilibrierung des Enzymsystems und des Netzwerkes eine Phosphat-Puffer-Lösung eingesetzt wird.

**Revendications**

1. Procédé de préparation de catalyseurs biologiques, en forme de perles, ayant une substance active enzymatiquement extrêmement sensible, par réticulation ionique de polycations en utilisant le mélange (A), sous la forme d'une solution ou d'une suspension, d'une substance active enzymatiquement dans la solution aqueuse d'un polyélectrolyte, caractérisé en ce qu'il consiste à introduire, dans le mélange (A), à titre de polyélectrolyte cationique, un chitosane protoné, ou un polymère naturel, ou un polymère naturel ayant subiune modification chimique, ou un polyélectrolyte cationique synthétique, dans une gamme de concentration de 0,1 à 15% en poids, et à utiliser, comme bain de précipitation, la solution d'un anion plurivalent dans la gamme de températures de —5°C à 80°C, comme mélange (B), et, au stade 1, à introduire le mélange (A) dans le mélange (B) par pulvérisation, avec formation de particules en forme de perles d'une granulométrie de 0,5 environ à 10 mm environ, et à solidifier celles-ci par agitation avec un temps de séjour de 5 minutes à 4 heures environ, et au stade 2, à séparer par filtration les perles de catalyseur formées avec la substance active enzymatiquement qui y est incluse, et, pour l'équilibrage, à les mettre dans une solution aqueuse stabilisant le système d'enzyme et le réseau, en ensuite à les sortir à l'état humide.

2. Procédé suivant la revendication 1, caractérisé en ce que le bain de précipitation (B) présente une gamme de concentration de 0,02 à 35%.

3. Procédé suivant les revendications 1 et 2, caractérisé en ce que la substance active enzymatiquement est constituée de cellules entières aptes à se reproduire, ou de cellules entières qui ne sont pas aptes à se reproduire, ou de fragments de cellules, et est mise en oeuvre sous la forme d'une suspension.

4. Procédé suivant les revendications 1 à 3, caractérisé en ce que la substance active enzymatiquement est constituée d'un enzyme isolé ou d'un complexe d'enzyme, et est mise en oeuvre sous la forme d'une solution ou d'une suspension.

5. Procédé suivant les revendications 1 à 4, caractérisé en ce qu'il consiste à utiliser comme substance active enzymatiquement des cellules, des fragments de cellules ou des enzymes, sensibles aux cations plurivalents ou sensibles mécaniquement, ou sensibles à la température, ou sensibles au séchage, tout en conservant des conditions osmotiques et physiologiques.

6. Procédé suivant les revendications 1 à 5, caractérisé en ce qu'il consiste à introduire dans le mélange (A), comme polyélectrolyte cationique, un polymère naturel, ou un polymère naturel ayant subi une modification chimique.

7. Procédé suivant les revendications 1 à 6, caractérisé en ce qu'il consiste à utiliser dans le mélange (A) un polyélectrolyte cationique synthétique.

8. Procédé suivant les revendications 1 à 7,

caractérisé en ce qu'il consiste à utiliser dans le mélange (A), comme polyélectrolyte cationique, un chitosane protoné en une gamme de concentration de 0,1 à 15% en poids.

9. Procédé suivant les revendications 1 à 8, caractérisé en ce qu'il consiste à utiliser dans le mélange (B), comme composé ionique à anion plurivalent,

$$K_4(Fe(CN)_6) \text{ et/ou } K_3(Fe(CN)_6)$$

en une gamme de concentration de 0,006 à 1 mole/l.

10. Procédé suivant les revendications 1 à 9, caractérisé en ce qu'il consiste à utiliser comme solution au stade 2, pour l'équilibrage du système d'enzyme et du réseau, une solution-tampon au phosphate.

## Claims

1. A method for producing bead-like biocatalysts with extreme enzymatically active substance by ionic network of polycations in using the mixture (A) as solution or suspension of an enzymatically active substance in the aqueous solution of a polyelectrode, characterized in that in mixture (A) a protonized chitosan or a natural polymer or a natural polymer after chemical modification or a synthetic cationic polyelectrolyte in a concentration range of 0,1 to 15% by weight is used as cationic polyelectroyte, and as a precipitation bath the solution of a polyvalent anion in a temperature range of —5°C to 80°C is used as mixture (B), and in stage 1 the mixture (A) being injected into the mixture (B), thus forming bead-like particles of a grain range of about 0,5 to about 10 mm, and said particles being compacted under stirring within a period of about 5 minutes to 4 hours, and in stage 2 the formed catalyst beads being filtered out with the entrapped enzymatically active substance, and for equilibration being introduced into an aqueous solution stabilizing the enzyme system and the network, and thereafter being discharged in the moist condition.

2. A method according to claim 1, characterized in that the precipitation bath (B) consists of a concentration range of 0,5 to 35% by weight.

3. A method according to claim 1 to 2, characterized in that the enzymatically active substance consists of whole cells capable of reproduction, whole cells incapable of reproduction or cell fragments, and is used in the form of a suspension.

4. A method according to claims 1 to 3, characterized in that the enzymatically active substance consists of an isolated enzyme or an enzyme-complex, and is used in the form of a solution or suspension.

5. A method according to claims 1 to 4, characterized in that sensitive or temperature-sensitive or drying-sensitive cells, cell fragments or enzymes under maintaining physiological, osmotical conditions are used as enzymatically active substance against polyvalent cations.

6. A method according to claims 1 to 5, characterized in that in the mixture (A) a natural polymer or a natural polymer after chemical modification is used as cationic polyelectrolyte.

7. A method according to claims 1 to 6, characterized in that in mixture (A) a synthetic cationic polyelectrolyte is used.

8. A method according to claims 1 to 7, characterized in that in mixture (A) a protonic chitosan in a concentration range of 0,1 to 15% by weight is used as cationic polyelectrolyte.

9. A method according to claims 1 to 8, characterized in that in mixture (B)

$$K_4(Fe(CN)_6) \text{ and/or } K_3(Fe(CN)_6)$$

in a concentration range of 0,006 to 1 mole/l (litre) is used as ionic compound with a polyvalent anion.

10. A method according to claims 1 to 9, characterized in that a phosphate buffer-solution is used as a solution in stage 2 for the equilibration of the enzyme system and of the network.